# EUROPÄISCHE PATENTANMELDUNG

(11) **EP 0 988 831 A1**
(43) Veröffentlichungstag der Anmeldung: **29.03.2000**
(21) Anmeldenummer: 99112295.3
(22) Anmeldetag: 26.06.1999
(51) Int. Cl.: A61B 17/04, A61B 17/28

(54) **Chirurgische Zange**

(30) Priorität: 23.09.1998 DE 19843577
(71) Anmelder: Aesculap AG & Co. KG, 78532 Tuttlingen (DE)
(72) Erfinder: Schwarz, Theodor, DE-78532 Tuttlingen (DE)
(74) Vertreter: Hoeger, Stellrecht & Partner

(57) **Zusammenfassung**

Um bei einer chirurgischen Zange (1) zum Greifen eines zylindrischen Stabes (16) mit zwei gegeneinander verschwenkbaren, jeweils eine Klemmbacke (5,6) tragenden Branchen (3,4), mit einer zur anderen Klemmbacke (5,6) hin offenen, quer zu den Klemmbacken (5) verlaufenden Einlegenut (9) für den Stab (16) und mit einer der Einlegenut (9) zugewandten Klemmfläche (13) an der anderen Klemmbacke (6), ein sicheres Greifen von Stäben (16) unterschiedlichen Durchmessers zu ermöglichen, wird vorgeschlagen, daß die Klemmfläche (13) in Längsrichtung der Einlegenut (9) ballig ausgebildet und in Richtung auf die Einlegenut (9) vorgewölbt ist.

## Beschreibung

Die Erfindung betrifft eine chirurgische Zange zum Greifen eines zylindrischen Stabes mit zwei gegeneinander verschwenkbaren, jeweils eine Klemmbacke tragenden Branchen, mit einer zur anderen Klemmbacke hin offenen, quer zu den Klemmbacken verlaufenden Einlegenut für den Stab und mit einer der Einlegenut zugewandten Klemmfläche an der anderen Klemmbacke.

Bei chirurgischen Operationen ist es oft notwendig, zylindrische Stäbe zu handhaben, beispielsweise Redon-Führungsnadeln, Haltestifte, etc., die mittels eines Werkzeuges in Längsrichtung in eine bestimmte Lage eingeschoben oder aus dieser Lage wieder herausgezogen werden müssen. Dabei sind unter Umständen erhebliche Kräfte aufzubringen, beispielsweise beim Durchstechen einer Redon-Führungsnadel durch die Bauchdecke.

Es ist Aufgabe der Erfindung, ein zangenförmiges Werkzeug zur Verfügung zu stellen, mit dem Stäbe unterschiedlichen Durchmessers so ergriffen werden können, daß sie im Werkzeug fixiert werden und daß mit dem Werkzeug große Kräfte in Längsrichtung des Stabes auf diesen übertragen werden können.

Diese Aufgabe wird bei einer chirurgischen Zange der eingangs beschriebenen Art erfindungsgemäß dadurch gelöst, daß die Klemmfläche in Längsrichtung der Einlegenut ballig ausgebildet und in Richtung auf die Einlegenut vorgewölbt ist.

Eine solche Ausgestaltung stellt sicher, daß der Stab nur an einer Stelle an der Klemmfläche sicher anliegt, so daß der Stab im Werkzeug in einer Dreipunktlage gehalten werden kann, nämlich an zwei Anlageflächen in der Einlegenut und an einer einzigen Stelle an der Klemmfläche. Dies ermöglicht unabhängig vom jeweiligen Durchmesser des Stabes ein sicheres Festlegen des Stabes in der Zange, so daß die Zange ohne weiteres für Stäbe mit unterschiedlichem Durchmesser verwendet werden kann, beispielsweise für Durchmesser zwischen 3 mm und 6 mm.

Insbesondere kann die Klemmfläche im Querschnitt kreisbogenförmig ausgebildet sein.

Es ist günstig, wenn die Klemmfläche quer zu der Einlegenut verlaufende nutförmige Vertiefungen aufweist, so daß zwischen diesen Vertiefungen rippenförmige Erhöhungen stehenbleiben, die quer zu den gehaltenen Stäben verlaufen und die dadurch die Anlageflächen weiter reduzieren.

Insbesondere gilt dies, wenn die Erhöhungen zwischen den nutförmigen Vertiefungen an ihrem höchsten Teil Anlegekanten ausbilden. Diese Kanten legen sich linienförmig an den Außenumfang des Stabes an und führen zu einer sehr hohen Flächenpressung und damit einem absolut sicheren Halt des Stabes in Längsrichtung.

Die Einlegenut und die Klemmflächen können genau senkrecht zu der Ebene verlaufen, die von den Klemmbacken aufgespannt wird, bei einer bevorzugten Ausführungsform ist jedoch vorgesehen, daß die Einlegenut und die Klemmfläche in einer Richtung verlaufen, die gegenüber einer durch die Klemmbacken aufgespannten Ebene einen Winkel zwischen 45° und 90° ausbildet.

Die Klemmbacken können in derselben Ebene liegen wie die Branchen, es ist aber bei einer bevorzugten Ausführungsform vorgesehen, daß die Klemmbacken in einer Ebene liegen, die gegenüber der Ebene der Branchen abgewinkelt ist. Insbesondere kann der Winkel zwischen der Ebene der Klemmbacken und der Ebene der Branchen zwischen 0° und 60° liegen.

Eine besonders günstige Ausgestaltung ergibt sich, wenn bei gegenüber den Branchen abgewinkelten Klemmbacken die Einlegenut und die Klemmfläche derart gegenüber der durch die Klemmbacken aufgespannten Ebene geneigt sind, daß die Längsrichtung der Einlegenut der von den Branchen aufgespannten Ebene angenähert ist. Beispielsweise könne die Klemmbacken in einer Ebene liegen, die gegenüber der Ebene der Branchen um 30° geneigt ist, die Einlegenut kann gegenüber der Ebene der Klemmbacken um 30° geneigt sein, und dann verläuft der Stab unter einem Winkel von etwa 30° gegenüber der Ebene der Branchen, so daß ein günstiger Angriffswinkel für das Vorschieben und Zurückziehen des Stabes gegeben ist.

Die Längsrichtung der Einlegenut kann dabei gegenüber der von den Branchen aufgespannten Ebene einen Winkel zwischen 15° und 60° einschließen.

Günstig ist es, wenn die Einlegenut im Querschnitt V-förmig ausgebildet ist, also durch zwei ebene, im Winkel zueinander stehende Seitenflächen begrenzt wird. Beim Einlegen eines im Querschnitt kreisförmigen Stabes legt sich dieser unabhängig von seinem eigenen Durchmesser immer linienförmig an diese Seitenflächen an und wird dann durch die punktförmige Anlage an der Klemmfläche kräftig in die Einlegenut hineingedrückt.

Bei einer bevorzugten Ausführungsform ist vorgesehen, daß die Klemmfläche zu ihrem von der Schwenkachse der Branchen weiter entfernten Ende hin in Richtung auf die gegenüberliegende Klemmbacke ansteigend ausgebildet ist. Die Klemmbacke hat dann also eine ansteigende Form, ihre Außenfläche ist zusätzlich ballig vorgewölbt.

Die Klemmfläche kann Teil eines Einsatzes sein, der in die Klemmbacke eingesetzt ist.

Es ist günstig, wenn den Branchen eine lösbare Sperre zugeordnet ist, die die Zange im geschlossenen Zustand festhält, in der zwischen den Klemmbacken ein Stab eingeklemmt ist.

Die nachfolgende Beschreibung bevorzugter Ausführungsformen der Erfindung dient im Zusammenhang mit der Zeichnung der näheren Erläuterung. Es zeigen:

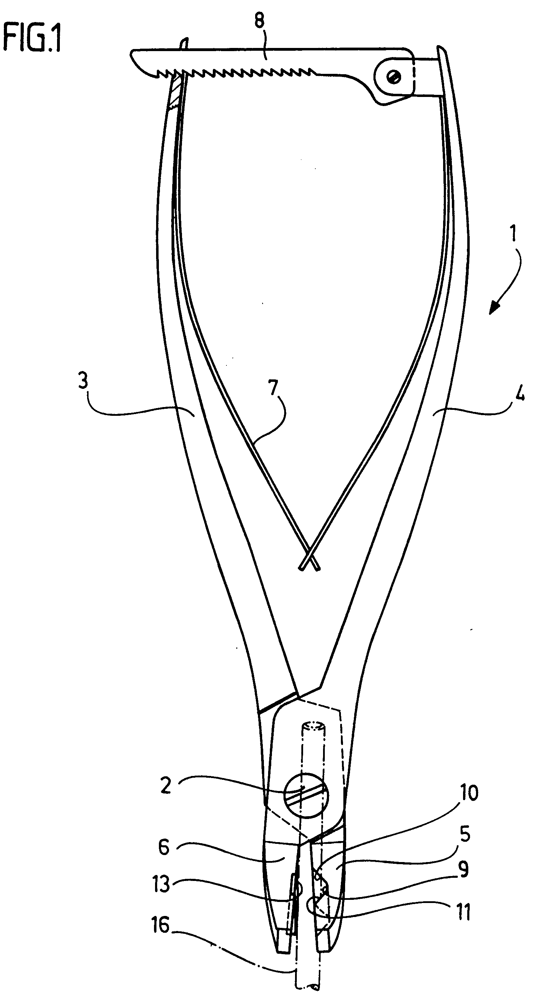

- Figur 1:: eine chirurgische Zange im geschlossenen Zustand mit einem eingespannten Stab;
- Figur 2:: eine vergrößerte Teilansicht der Zange der Figur 1 im Bereich der Klemmbacken mit einem zwischen den Klemmbacken eingelegten dünnen Stab;
- Figur 3:: eine Schnittansicht längs Linie 3-3 in Figur 2;
- Figur 4:: eine Schnittansicht längs Linie 4-4 in Figur 3;
- Figur 5:: eine Ansicht ähnlich Figur 2 mit einem zwischen die Klemmbacken eingelegten dickeren Stab und
- Figur 6:: eine Schnittansicht längs Linie 6-6 in Figur 5.

Die in der Zeichnung dargestellte Zange 1 umfaßt zwei um eine Schwenkachse 2 schwenkbar miteinander verbundene Branchen 3, 4, von denen jede einen Klemmbacken 5, 6 trägt. Die beiden Branchen 3 und 4 werden durch ein an ihnen gehaltenes Federelement 7 auseinandergedrückt, am hinteren Ende der Branchen 3, 4 ist eine Zahnsperre 8 gelagert, die die Branchen 3, 4 in bestimmten Zwischenlagen festlegt und die durch Verschwenken gelöst werden kann, so daß dann die Branchen 3 und 4 unter der Wirkung des Federelementes 7 auseinandergeschwenkt werden.

Die beiden Klemmbacken 5 und 6 spannen eine Ebene auf, die gegenüber der Ebene, die durch die Branchen 3 und 4 aufgespannt wird, geneigt ist, beispielsweise um einen Winkel von 30°.

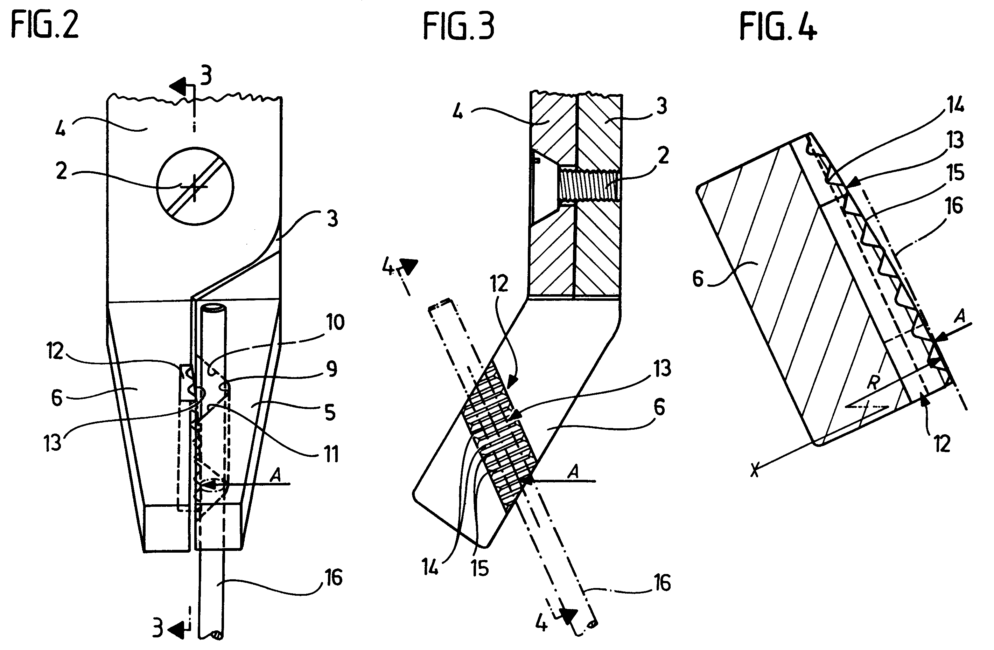

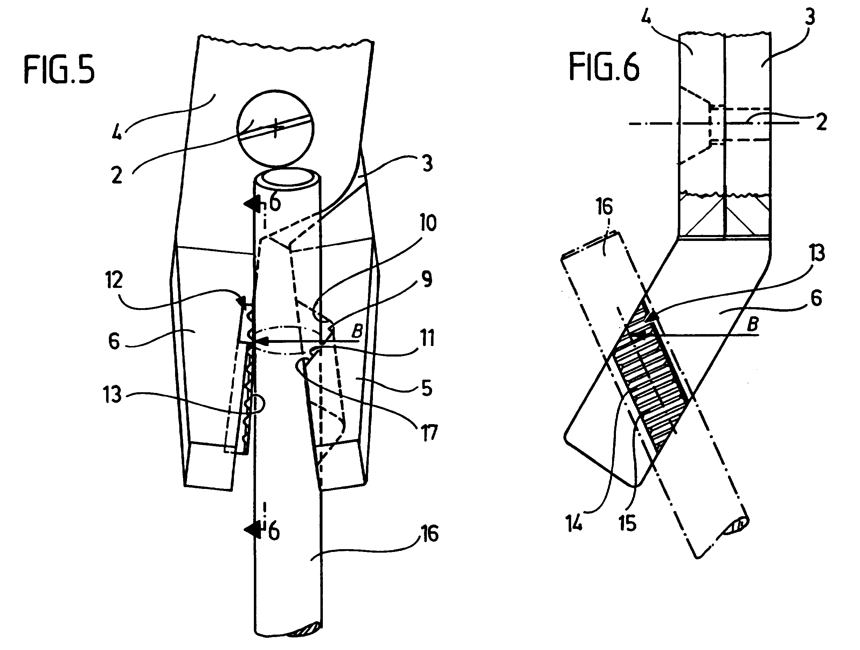

Die Klemmbacke 5 trägt an ihrer Innenseite eine im Querschnitt V-förmige Einlegenut 9, die zur anderen Klemmbacke 6 hin offen ist und die gegenüber der von den Klemmbacken 5 und 6 aufgespannten Ebene um einen Winkel von etwa 60° geneigt ist, so daß deren Längsrichtung zu der von den Branchen aufgespannten Ebene etwa einen Winkel von 30° einschließt (Figuren 3 und 6).

Die Einlegenut 9 weist zwei ebene Seitenflächen 10, 11 auf, der Winkel zwischen ihnen beträgt etwa 90°.

Der Einlegenut 9 gegenüber und mit dieser ausgerichtet ist in der anderen Klemmbacke 6 ein Einsatz 12 eingesetzt, der der Einlegenut 9 gegenüberliegend eine Klemmfläche 13 ausbildet. Diese Klemmfläche 13 ist mit quer zur Längsrichtung der Einlegenut 9 verlaufenden, rinnen- oder nutförmigen Vertiefungen 14 versehen, zwischen denen rippenförmige Vorsprünge 15 ausgebildet sind, die an ihren freien Enden kantenförmig auslaufen und dort linienförmige Anlagebereiche ausbilden. Die Hüllkurve dieser Vorsprünge 15 ist in Längsrichtung der Einlegenut 9 kreisbogenförmig geformt und zur Einlegenut 9 vorgewölbt, der Radius des Bogens kann beispielsweise zwischen 10 und 20 cm liegen. Außerdem ist der Einsatz 12 keilförmig ausgebildet, seine Dicke nimmt mit größer werdendem Abstand von der Schwenkachse 2 zu, so daß insgesamt eine zur Einlegenut 9 gewölbte und zum vorderen Ende der Klemmbacken geringfügig vorspringende Klemmfläche 13 entsteht.

Die in dieser Weise ausgestaltete Zange kann kreiszylindrische Stäbe 16 unterschiedlichen Durchmessers festhalten. Diese werden in die Einlegenut 9 derart eingelegt, daß sie sich linienförmig an die beiden Seitenflächen 10 und 11 der Einlegenut 9 anlegen, beim Schließen der Branchen 3 und 4 legt sich außerdem die Klemmfläche 13 an den Stab 16 an und preßt diesen kräftig in die Einlegenut 9 hinein, in dieser Stellung der Branchen 3 und 4 werden diese durch die Zahnsperre 8 gehalten.

Aufgrund der speziellen Ausgestaltung der Klemmfläche 13 legt sich diese nicht flächig an den eingelegten Stab 16 an, sondern punktförmig. Dies ergibt sich durch die gewölbte Ausgestaltung der Klemmfläche 13, diese Wölbung stellt sicher, daß auch bei unterschiedlichen Durchmessern des Stabes 16 und damit bei unterschiedlichem Öffnungswinkel der Klemmbacken 5 und 6 in geschlossenem Zustand immer nur ein Punkt der gewölbten Klemmfläche 13 an dem Stab 16 zur Anlage kommt, diese Lokalisierung der Anlagefläche wird noch dadurch unterstützt, daß die Klemmfläche 13 quer zur Längsrichtung der Stäbe verlaufende, rippenförmige Vorsprünge 15 trägt, die sich mit ihren Kanten in den Stab 16 mit erhöhter Flächenpressung eindrücken können und dadurch den Stab 16 zwischen den Klemmbacken 5 und 6 gegen jede Längsverschiebung sichern können.

Die beschriebene Zange ist daher geeignet, Stäbe 16 unterschiedlichen Durchmessers festzulegen, wie dies auch in der Zeichnung dargestellt ist. Bei dem Beispiel der Figuren 2 bis 4 wird ein dünner Stab 16 ergriffen, die Klemmfläche 13 legt sich in diesem Falle mit einem Teilbereich A an den Stab an, der von der Schwenkachse 2 weit entfernt ist.

Bei einem dickeren Stab 16, wie er in den Figuren 5 und 6 dargestellt ist, ist der Anlagebereich B der Klemmfläche 13 in Richtung auf die Schwenkachse 2 verschoben, in allen anderen Bereichen ergibt sich kein Kontakt zwischen Klemmfläche 13 und Stab 16.

Insbesondere bei dickeren Stäben ist es auch möglich, daß der Stab zum vorderen Ende der Zange hin aus der Einlegenut 9 herausgeschwenkt wird, so daß er nur im hintersten Bereich der Einlegenut 9 an den Seitenflächen 10 und 11 anliegt, also im Bereich der Austrittskante 17 der Einlegenut 9. Der Stab ist somit zwischen dieser Austrittskante 17 einerseits und dem Anlagepunkt B der Klemmfläche 13 andererseits eingespannt und in Längsrichtung zuverlässig gehalten.

## Patentansprüche

1. Chirurgische Zange zum Greifen eines zylindrischen Stabes mit zwei gegeneinander verschwenkbaren, jeweils eine Klemmbacke tragenden Branchen, mit einer zur anderen Klemmbacke hin offenen, quer zu den Klemmbacken verlaufenden Einlegenut für den Stab und mit einer der Einlegenut zugewandten Klemmfläche an der anderen Klemmbacke, dadurch gekennzeichnet, daß die Klemmfläche (13) in Längsrichtung der Einlegenut (9) ballig ausgebildet und in Richtung auf die Einlegenut (9) vorgewölbt ist.

2. Zange nach Anspruch 1, dadurch gekennzeichnet, daß die Klemmfläche (13) im Querschnitt kreisbogenförmig ausgebildet ist.

3. Zange nach einem der voranstehenden Ansprüche, dadurch gekennzeichnet, daß die Klemmfläche (13) quer zu der Einlegenut (9) verlaufende, nutförmige Vertiefungen (14) aufweist.

4. Zange nach Anspruch 3, dadurch gekennzeichnet, daß die Erhöhungen (15) zwischen den nutförmigen Vertiefungen (14) an ihrem höchsten Teil Anlegekanten ausbilden.

5. Zange nach einem der voranstehenden Ansprüche, dadurch gekennzeichnet, daß die Einlegenut (9) und die Klemmfläche (13) in einer Richtung verlaufen, die gegenüber einer durch die Klemmbacken (5, 6) aufgespannten Ebene einen Winkel zwischen 45° und 90° ausbildet.

6. Zange nach einem der voranstehenden Ansprüche, dadurch gekennzeichnet, daß die Klemmbacken (5, 6) in einer Ebene liegen, die gegenüber der Ebene der Branchen (3, 4) abgewinkelt ist.

7. Zange nach Anspruch 6, dadurch gekennzeichnet, daß der Winkel zwischen der Ebene der Klemmbacken (5, 6) und der Ebene der Branchen (3, 4) zwischen 0 und 60° liegt.

8. Zange nach Anspruch 7, dadurch gekennzeichnet, daß bei gegenüber den Branchen (3, 4) abgewinkelten Klemmbacken (5, 6) die Einlegenut (9) und die Klemmfläche (13) derart gegenüber der durch die Klemmbacken (5, 6) aufgespannten Ebene geneigt sind, daß die Längsrichtung der Einlegenut (9) der von den Branchen (3, 4) aufgespannten Ebene angenähert ist.

9. Zange nach Anspruch 8, dadurch gekennzeichnet, daß die Längsrichtung der Einlegenut (9) gegenüber der von den Branchen (3, 4) aufgespannten Ebene einen Winkel zwischen 15° und 60° einschließt.

10. Zange nach einem der voranstehenden Ansprüche, dadurch gekennzeichnet, daß die Einlegenut (9) im Querschnitt V-förmig ausgebildet ist.

11. Zange nach einem der voranstehenden Ansprüche, dadurch gekennzeichnet, daß die Klemmfläche (13) zu ihrem von der Schwenkachse (2) der Branchen (3, 4) weiter entfernten Ende hin in Richtung auf die gegenüberliegende Klemmbacke (5) ansteigend ausgebildet ist.

12. Zange nach einem der voranstehenden Ansprüche, dadurch gekennzeichnet, daß den Branchen (3, 4) eine lösbare Sperre (8) zugeordnet ist.
